# EUROPÄISCHE PATENTSCHRIFT

(11) **EP 2 151 197 B1**
(45) Veröffentlichungstag und Bekanntmachung des Hinweises auf die Patenterteilung: **28.03.2012**
(21) Anmeldenummer: 09167087.7
(22) Anmeldetag: 03.08.2009
(51) Int. Cl.: A61B 17/02

(54) **Schnellspannklemme zur Anbringung von chirurgischen Hilfmitteln wie Wundhaken oder peripherer Operationstechniken an ein Rahmensystem**
Quick tensioning clamp for attaching surgical aids such as retractors or peripheral operation technology on a frame system
Pince de serrage rapide destinée à l'application de moyens d'aide chirurgicaux comme un écarteur pour plaies ou des techniques d'opération périphériques sur un système de cadre

(30) Priorität: 06.08.2008 DE 102008036673
(43) Veröffentlichungstag der Anmeldung: 10.02.2010
(73) Patentinhaber: Medizintechnik Sattler GmbH, 07426 Königsee/Thür. (DE)
(72) Erfinder: Sattler, Sven, 07426, Königsee (DE)
(74) Vertreter: Kruspig, Volkmar

(56) Entgegenhaltungen:
- EP-A- 0 972 491
- EP-A- 1 683 488
- US-A1- 2002 172 549
- US-A1- 2003 199 738
- US-B1- 7 297 107

## Beschreibung

Die Erfindung betrifft eine Schnellspannklemme zur Anbringung von chirurgischen Hilfsmitteln wie Wundhaken oder peripherer Operationstechnik an ein Rahmensystem, gemäß Oberbegriff des Patentanspruchs 1.

Eine Reihe von Operationen müssen mit einem stark vergrößerten Operationsfeld durchgeführt werden. Um derartige Operationsfelder zu erhalten, müssen Hautlappen und Gewebe mit Wundhaken zurückgehalten werden, um dem Operateur freie Sicht auf die geöffneten Körperhöhlen zu ermöglichen. Wundhaken werden dabei oftmals von OP-Schwestern oder Assistenzärzten im Verlaufe der Operation gehalten.

Bei lang andauernde Operationen stellt dies eine sehr starke körperliche Belastung dar, zumal nicht verhindert werden kann, dass eventuelle kleine Bewegungen des OP-Personals auf den Wundhaken und somit auf das Gewebe übertragen werden.

Aufgrund dessen werden bei Operationen im zunehmenden Maße sogenannte Retraktionssysteme verwendet. Retraktionssysteme bestehen aus einem variabel zusammensetzbaren Metallrahmen an dessen Verbindungsstangen chirurgische Hilfsmittel wie Wundhaken oder periphere Operationstechnik befestigt werden. Zur Anbringung der chirurgischen Hilfsmittel an das Rahmensystem werden Klemmschrauben verwendet.

Aus der DE 697 35 758 T2 ist ein Haltegelenk zur Befestigung chirurgischer Hilfsmittel an ein Retraktionssystem bekannt, das zwei Halteelemente aufweist. Das erste Halteelement ist dabei um die Achse eines Schaftes drehbar gelagert und an ein Nockenelement gekoppelt. Abhängig von der jeweiligen Klemmposition des Gelenks kontrolliert das Nockenelement die Drehbewegung des ersten Halteelements. Zusätzlich umfasst das Gelenk ein zweites Halteelement, das dem ersten Halteelement gleicht. Dadurch kann beispielsweise mittels des erstens Halteelements die Verbindung zum Retraktionssystems hergestellt werden und mittels des zweiten Halteelements das chirurgische Hilfsmittel an dem Retraktionssystem befestigt werden.

Die Halteelemente weisen jeweils zwei Schenkelelemente auf, die eine Klemmbohrung begrenzen. Die zu verbindenden chirurgischen Hilfselemente oder Verbindungsstangen des Retraktionssystems müssen folglich in die Klemmbohrungen eingeführt werden, wo sie aufgrund des zu betätigenden Nockenmechanismus festgeklemmt werden.

Müssen beispielsweise mehrere chirurgische Hilfsmittel an einer Retraktionsverbindungsstange befestigt werden, müssen die zugehörigen Haltegelenke nacheinander in der richtigen Reihenfolge auf die Stange gefädelt werden. Im Laufe einer Operation ist es jedoch oftmals erforderlich, chirurgische Hilfselemente zu entfernen und durch andere zu ersetzten. Mit dem soeben beschriebenen Haltegelenk führt dies eine erhebliche Umbaumaßnahme des Retraktionssystems mit sich, währenddessen keine stabile Halterung aller Hilfsmittel und gleichbleibende Spannung des zurückhaltenden Gewebes gewährleistet werden kann.

Außerdem zeigt die Anwendung des beschrieben Haltegelenks in der Praxis, dass blutverschmierte Blatthalter nicht mehr in die passgenauen Haltelemente eingeführt werden können, sondern zuvor gereinigt werden müssen. Auch dies zieht einen erheblichen zeitlichen bzw. materiellen Aufwand nach sich.

Auch aus den Druckschriften EP 0 972 491 A1, US 2003/0199738 A1 und US 7,2797,107 B sind Vorrichtungen zu Fixierung eines Hilfsmittels an eine Rahmenstange bekannt, wobei mindestens ein als Klemmbohrung ausgeführtes Klemmelement vorhanden ist.

Aus dem Vorgenannten ist es daher Aufgabe der Erfindung eine weiterentwickelte Klemmvorrichtung anzugeben, welche eine schnelle, variable und über die vollständige Operationsdauer hinweg sichere Verbindung chirurgischer Hilfselemente mit einem Retraktionssystem mit gleichzeitiger Reduzierung der Anzahl der mechanischen Einzelteile der Klemmvorrichtung ermöglicht.

Die Lösung der Aufgabe der Erfindung erfolgt durch eine Schnellspannklemme zur Anbringung von chirurgischen Hilfsmitteln wie Wundhaken oder peripherer Operationstechnik an ein Rahmensystem, gemäß Oberbegriff des Patentanspruchs 1, wobei die Unteransprüche mindestens zweckmäßige Ausgestaltungen und Weiterbildungen darstellen.

Die Schnellspannklemme umfasst zwei mit einer zentralen Aufnahme für einen Zugbolzen versehene Klemmelemente, wobei das mit dem Rahmensystem zu verbindende Klemmelement zwei miteinander verbundene Schenkel aufweist. Die beiden Klemmelemente stehen dabei mittels eines Zugbolzens, der durch in den Klemmelementen eingearbeitete Durchtrittsöffnungen geführt ist, miteinander in mechanischer Wirkung, wobei an dem zum ersten Klemmelement gegenüberliegenden Ende der Zugbolzen ein Exzenterhebel angeordnet ist.

Erfindungsgemäß ist das zweite Klemmelement als einteiliger Schenkel ausgeführt, dessen Querschnitt eine viertelkreisförmige Aussparung aufweist, wobei der Gegenschenkel durch einen äußeren Oberflächenabschnitt eines der Schenkel des ersten Klemmelements gebildet ist. Das zweite Klemmelement nimmt ein an das Rahmensystem zu verbindendes chirurgisches Hilfsmittel auf.

Zweckmäßigerweise sind die Durchtrittsöffnungen in der Schenkelbasis des jeweiligen Klemmelements eingearbeitet und an die jeweilige geometrische Form des entsprechenden Abschnitts des Zugbolzens angepasst, um diesen durch die beiden Klemmelemente führen zu können.

Das mit dem ersten Klemmelement in Verbindung stehende Ende des Zugbolzens ist mit einer, den Durchmesser einer um die ursprüngliche Durchtrittsöffnung des Klemmelements vergrößernden Senkung entsprechend minimal kleiner gewählten Verdickung versehen, sodass das Hineinrutschen des Zugbolzens in das erste Klemmelement verhindert wird. Das erste Klemmelement ist dabei im nichtbetätigten Zustand, das heißt mit geöffnetem Klemmhebel, drehbar um die Achse des Zugbolzens gelagert.

Zweckmäßigerweise ist der Zugbolzen im Bereich des zweiten Klemmelements der Geometrie der Durchtrittsöffnung des zweiten Klemmelements angepasst, wobei die Maße des Zugbolzens und der Durchtrittsöffnung in diesem Bereich derartig gewählt werden, sodass das zweite Klemmelement und der Zugbolzen eine Einpressverbindung eingehen. Das zweite Klemmelement dreht sich folglich mit dem betätigten Zugbolzen mit und ist nicht wie das erste Klemmelement drehbar gelagert.

Aufgrund dieser Konstruktion können chirurgische Hilfsmittel in unterschiedlichsten Positionen und Winkelstellungen an einem Rahmensystem befestigt werden. Der geringe Abstand bzgl. des anzubringenden Hilfsmittels zum Rahmen und der geringe Überstand der Konstruktion im Operationsfeld ermöglichen dem Operateur einen größeren Bewegungsspielraum.

An dem zum zweiten Klemmelement gerichteten Ende des Zugbolzens ist mittels eines Querbolzens ein Exzenterhebel angebracht, wobei der Querbolzen in einer besonders bevorzugten Ausführungsform aus Bronze gefertigt ist.

Der Exzenterhebel erlaubt bei geringstem Kraftaufwand eine möglichst große Kraftwirkung auf die erfinderische Schnellspannklemme, sodass eine Betätigung der Vorrichtung mit nur einer Hand möglich ist. Vorzugsweise weist der Exzenterhebel mindestens eine Bohrung auf einer Hebelfläche auf. Eine derartige Ausführung ist vor allem aus Gründen der Gewichtseinsparung und verringerten Blendwirkung von Vorteil.

Außerdem ist die Schnellspannklemme so konstruiert, dass der Exzenterhebel aus wahlweisen Richtungen betätigt werden kann und in wahlweisen Richtungen während des "geklemmten Zustandes" ragen kann. Um den Operateur während der Operation mehr Bewegungsfreiheit zu gewährleisten und zu vermeiden, dass sich der Operateur im Bauchbereich am Exzenter stößt, kann es von Vorteil sein; den Exzenterhebel in Richtung der geöffneten Körperhöhle bzw. Wunde ragen zu lassen.

Der Exzenterhebel wird im Allgemeinen aus der Blickrichtung des Operateurs auf die Wunde von oben betätigt.

Um die Hebelkraft des betätigten Exzenterhebels auf das zweite Klemmelement zu übertragen, ist zwischen dem zweiten Klemmelement und dem Exzenterhebel ein Druckstück angebracht, welches in eine Aussparung an der Oberseite des zweiten Klemmelements gedrückt ist. In einer besonders bevorzugten Ausführungsform ist das Druckstück aus Polyetheretherketon gefertigt.

Um den Zugbolzen auch durch das Druckstück führen zu können, muss dieses eine dem Zugbolzen entsprechende Durchtrittsöffnung aufweisen.

Zwischen dem Druckstück und dem zweiten Klemmelement ist in der Aussparung ein federnder O-Ring eingebracht, der vorzugsweise aus medizinischem Silikon gefertigt ist. Dieses Material lässt sich besonders gut reinigen und sorgt aufgrund einer wirkenden Vorspannung dafür, dass der im zweiten Klemmelement eingeklemmte Blatthalter auch bei geöffnetem Exzenterhebel nicht herausrutscht und die Konstruktion weiterhin um 360 ° drehbar ist.

Außerdem ist auf der zum zweiten Klemmelement gerichteten Oberseite des ersten Klemmelements ein in eine Aussparung auf der Oberseite eingebrachtes Gleitlager vorgesehen, wobei es sich vorzugsweise um ein Peek-Gleitlager handelt. Das Gleitlager verhindert einen schnellen Verschleiß und erhöht die Verwendungsdauer des Klemmelements. Der Durchmesser des Gleitlagers entspricht im Wesentlichen den Querschnitts-Abmaßen des zweiten Klemmelements.

Die Schenkel des ersten Klemmelements begrenzen bei der erfindungsgemäßen Schnellspannklemme eine halbkreisförmige Aussparung, welche dem Durchmesser von Rahmenstangen des zu verbindenden Rahmensystems entspricht, wobei der Durchmesser der halbkreisförmigen Aussparung etwa 10 % größer als der Durchmesser der Rahmenstange gewählt wird.
Es ist vorgesehen die Klemmelemente zunächst als Halbfertigprodukte zu fertigen, die aufgrund der Bestellanforderungen der Kunden und den vorhandenen Rahmensystemgrößen zusammengesetzt werden.

Erfindungsgemäß weisen die Schenkel des ersten Klemmelements an den von der Schenkelbasis weggerichteten Enden innenseitig Abflachungen auf, sodass das erste Klemmelement mit sehr geringem Kraftaufwand auf die Rahmenstange geschoben bzw. geklickt werden kann.

Um dem ersten Klemmelement die benötigte elastische Verformbarkeit zur Ausführung des Klickvorgangs zu verleihen, weist das Klemmelement im Bereich der Schenkelbasis eine von der halbkreisförmigen Aussparung weggerichtete schlitzförmige Aussparung auf, welche wiederum in einer Vollbohrung endet.

Aufgrund des dargelegten Klickmechanismus können die Klemmelemente schnell vom Rahmensystem entfernt werden. Dies erspart bei einem gewünschten Austausch bzw. der Entfernung einzelner chirurgischer Hilfsmittel das einzelne Aus- bzw. Einfädeln aller Klemmelemente auf das Rahmensystem.

Außerdem wirken sich eventuelle Blutablagerung auf Blatthaltern oder ähnlichen Konstruktionen nicht mehr negativ auf die auszuführenden Klemmvorgänge aus, da das zweite Klemmelement aufgrund der Ausbildung als einteiliger Schenkel ausreichend Spielraum zur Fixierung von Blatthaltern mit zusätzlichen, Durchmesser-vergrößernden Ablagerungen aufweist.

Die Erfindung soll nachstehend anhand eines Ausführungsbeispiels sowie unter Zuhilfenahme von Figuren näher erläutert werden.

Hierbei zeigen:
- Fig. 1: eine Darstellung der erfindungsgemäßen Schnellspannklemme;
- Fig. 2: eine Explosionsdarstellung aller Einzelteile der Schnellspannklemme und
- Fig. 3: eine an ein Rahmensystem geklickte Schnellspannklemme mit fixier- tem Blatthalter.

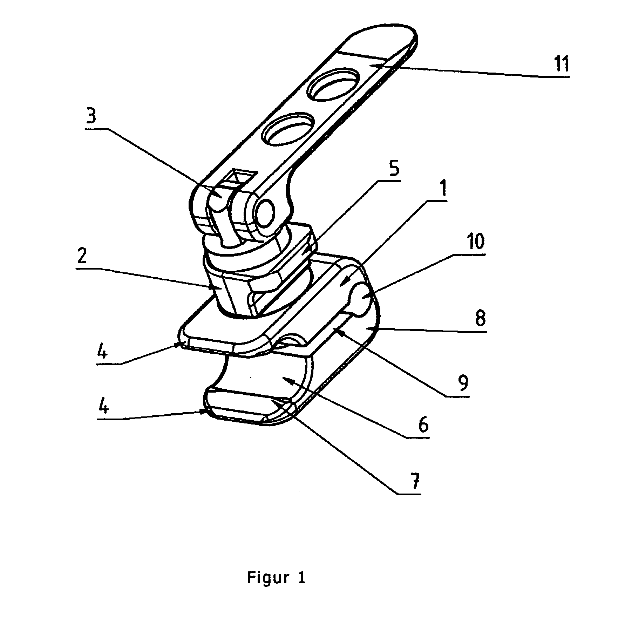

Die in Fig. 1 dargestellte Schnellspannklemme umfasst ein erstes Klemmelement 1 und ein zweites Klemmelement 2, welche eine zentrale Aufnahme für einen Zugbolzen 3 aufweisen. Das erste Klemmelement 1 weist dabei zwei miteinander verbundene Schenkel 4 auf. Das zweite Klemmelement 2 ist hingegen nur als einteiliger Schenkel 5 ausgebildet. Der Gegenschenkel des zweiten Klemmelements 2 wird durch einen äußeren Oberflächenabschnitt eines der Schenkel 4 des ersten Klemmelements 1 gebildet.

Vorzugsweise wird die Schnellspannklemme mit dem ersten Klemmelement 1 an die Verbindungsstangen eines Rahmensystems geklickt und das zweite Klemmelement 2 dient zur Aufnahme eines chirurgischen Hilfsmittels.

Die beiden Schenkel 4 des ersten Klemmelements 1 begrenzen eine halbkreisförmige Aussparung 6, die dem Durchmesser von Verbindungsstangen des zu verbindenden Rahmensystems entspricht. An der Innenseite der Schenkel 4 des ersten Klemmelements 1 sind Abflachungen 7 vorgesehen, um das Aufklicken der Schnellspannklemme auf ein Rahmensystem zu erleichtern. Um dem ersten Klemmelement 1 eine zusätzliche Elastizität zur Ausführung des Klickvorgangs zu verleihen, ist in der Schenkelbasis 8 des ersten Klemmelements 1 eine schlitzförmige Aussparung 9 vorgesehen, welche von der halbkreisförmigen Aussparung 6 weggerichtet ist und in einer Vollbohrung 10 endet.

Die Fixierung eines chirurgischen Hilfsmittels wie z. B. eines Wundhakens erfolgt durch die Betätigung des Exzenterhebels 11. Bei senkrechter Position des Exzenterhebels wird das chirurgische Hilfsmittel zwischen den Schenkel 5 des zweiten Klemmelements und einem Oberflächenabschnitt des ersten Klemmelements 1 positioniert. Anschließend kann der Exzenterhebel 11 in waagrechte Position gebracht werden, was eine Fixierung des chirurgischen Hilfsmittels nach sich zieht.

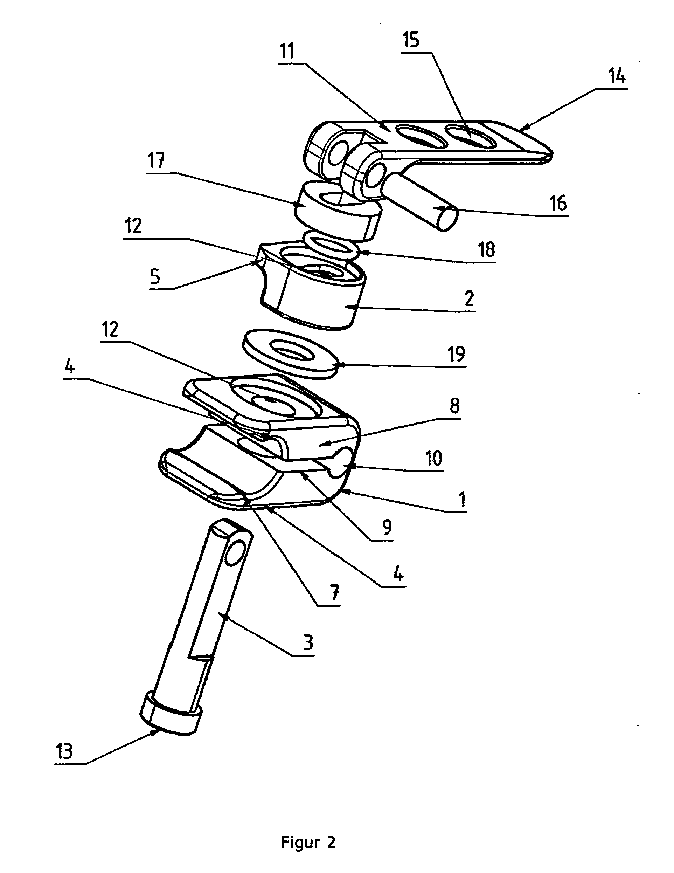

Die in Fig. 2 dargestellten Einzelteile der erfindungsgemäßen Schnellspannklemme verdeutlichen das Wirkungsprinzip derselbigen. Der Zugbolzen 3 wird durch die Durchtrittsöffnungen 12 der Klemmelemente (1, 2) geführt. Wobei das mit dem ersten Klemmelement 1 in Verbindung stehende Ende des Zugbolzens 3 eine Verdickung 13 aufweist, dessen Abmaße minimal kleiner als der Durchmesser der Senkung gewählt sind, welche den Durchmesser der ursprünglichen Durchtrittsöffnung 12 vergrößert. Dadurch kann der Zugbolzen 3 nicht in Richtung des zweiten Klemmelements 2 rutschen.

Außerdem weist der Zugbolzen 3 im Bereich des zweiten Klemmelements 2 Abflachungen auf, sodass die Geometrie der Durchtrittsöffnung 12 des zweiten Klemmelements nachgebildet wird. Die Abmaße der Abflachungen und der zugehörigen Durchtrittsöffnung 12 sind so gewählt, dass das zweite Klemmelement 2 mit dem Zugbolzen 3 eine Einpressverbindung eingeht. Das zweite Klemmelement 2 folgt also den rotatorischen Bewegungen des Zugbolzens 3, währenddessen das erste Klemmelement 1 um die Achse des Zugbolzens 3 drehbar gelagert ist.
Bei Betätigung des Exzenterhebels 11, der aus einer Hebelfläche 14 mit eingearbeiteten Bohrungen 15 besteht und mittels eines Querbolzens 16 mit dem Zugbolzen 3 verbunden ist, wird entsprechende Kraft auf ein Druckstück 17 übertragen. Dieses Druckstück 17 ist in eine Aussparung auf der Oberfläche des zweiten Klemmelements 2 eingebracht und drückt folglich das zweite Klemmelement 2 in Richtung des ersten Klemmelements 1. Das Druckstück 17 ist vorzugweise aus Polyetheretherketon gefertigt.

In die Aussparung auf der Oberfläche des zweiten Klemmelements 2 ist ein zusätzlicher O-Ring 18 eingebracht, der die federnde Wirkung auf das zweite Klemmelement 2 unterstützt. Der O-Ring 18 ist beispielsweise aus Silikon hergestellt.

Zusätzlich wird in eine Aussparung auf der zum zweiten Klemmelement 2 zeigenden Oberfläche des ersten Klemmelements 1 ein Gleitlager 19 eingesetzt. Dieses schützt die Schnellspannklemme vor großem Abrieb, der bei jeder Betätigung des Exzenterhebels 11 und der darauffolgenden Reibung des zweiten Klemmelements 2 am ersten Klemmelement 1.

Da das zweite Klemmelement 2 als einteiliger Schenkel 5 ausgebildet ist, weist die gesamte Schnellspannklemme eine relativ geringe Bauhöhe auf. Aufgrund dessen und der drehbaren Lagerung des ersten Klemmelements 1 um die Achse des Zugbolzens 3 können chirurgische Hilfsmittel in unterschiedlichsten Positionen und Winkelstellungen an einem Rahmensystem befestigt werden. Der geringe Abstand bzgl. des anzubringenden Hilfsmittels zum Rahmen und der geringe Überstand der Konstruktion im Operationsfeld ermöglichen dem Operateur einen größeren Bewegungsspielraum.

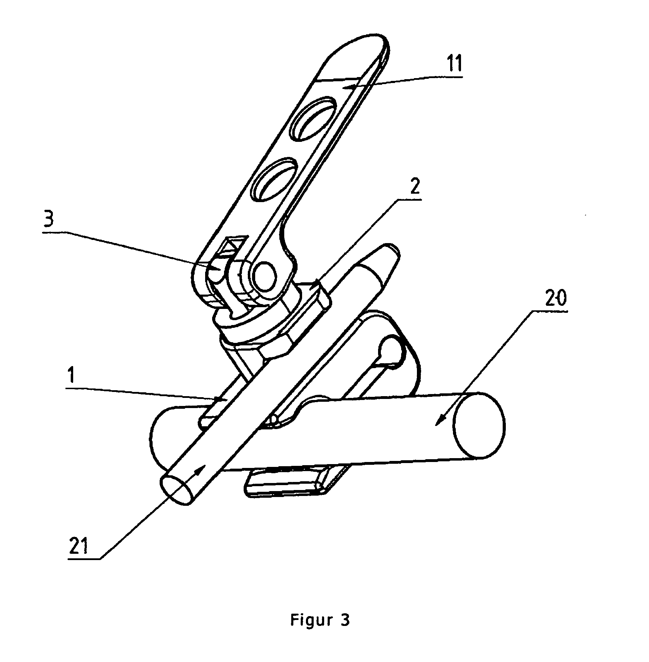

Die Fig. 3 zeigt die Schnellspannklemme im eingebauten Zustand. Dabei ist das erste Klemmelement 1 auf eine Verbindungsstange 20 des Rahmensystems geschoben, währenddessen das zweite Klemmelement 2 einen Blatthalter 21 fixiert.

### Bezugszeichenliste

- 1: Erstes Klemmelement
- 2: Zweites Klemmelement
- 3: Zugbolzen
- 4: Schenkel des ersten Klemmelements
- 5: Schenkel des zweiten Klemmelements
- 6: Halbkreisförmige Aussparung
- 7: Abflachungen
- 8: Schenkelbasis
- 9: Schlitzförmige Aussparung
- 10: Vollbohrung
- 11: Exzenterhebel
- 12: Durchtrittsöffnung
- 13: Verdickung
- 14: Hebelfläche
- 15: Bohrung
- 16: Querbolzen
- 17: Druckstück
- 18: O-Ring
- 19: Gleitlager
- 20: Verbindungsstange
- 21: Blatthalter

## Patentansprüche

1. Schnellspannklemme zur Anbringung von chirurgischen Hilfsmitteln wie Wundhaken oder peripherer Operationstechnik an ein Rahmensystem, umfassend zwei mit einer zentralen Aufnahme für einen Zugbolzen (3) versehene Klemmelemente (1; 2), wobei
das erste Klemmelement (1) um die Achse des Zugbolzens (3) drehbar gelagert ist und
die beiden Klemmelemente (1; 2) mittels des Zugbolzens (3), der durch in den Klemmelementen (1; 2) eingearbeitete Durchtrittsöffnungen (12) geführt ist, miteinander in mechanischer zueinander drehbaren Wirkung stehen und
an dem zum ersten Klemmelement (1) gegenüberliegenden Ende des Zugbolzens (3) mittels eines Querbolzens (16) ein Exzenterhebel angebracht ist,
wobei das mit dem Rahmensystem zu verbindende erste Klemmelement (1) zwei miteinander verbundene Schenkel (4) aufweist,
**dadurch gekennzeichnet, dass**
das zweite Klemmelement (2) als einteiliger Schenkel (5), dessen Querschnitt eine viertelkreisförmige Aussparung aufweist, ausgeführt ist und der Gegenschenkel durch einen äußeren Oberflächenabschnitt eines der Schenkel (4) des ersten Klemmelements (1) gebildet ist,
und auf der zum zweiten Klemmelement (2) gerichteten Oberseite des ersten Klemmelements (1) ein in eine Aussparung auf der Oberseite eingebrachtes Gleitlager (19) vorgesehen ist, wobei der Durchmesser des Gleitlagers (19) im Wesentlichen den Querschnitts-Abmaßen des zweiten Klemmelements (2) entspricht und
zwischen dem zweiten Klemmelement (2) und dem Exzenterebel (11) ein Druckstück (17) angebracht ist, welches in eine Aussparung an der Oberseite des zweiten Klemmelements (2) gedrückt ist und zwischen dem Druckstück (17) und dem zweiten Klemmelement (2) in die Aussparung ein federnder O-Ring (18) eingebracht ist.

2. Schnellspannklemme nach Anspruch 1,
**dadurch gekennzeichnet, dass**
die Durchtrittsöffnungen (14) in der Schenkelbasis (8) des jeweiligen Klemmelements (1; 2) eingearbeitet sind.

3. Schnellspannklemme nach Anspruch 1 oder 2,
**dadurch gekennzeichnet, dass**
das mit dem ersten Klemmelement (1) in Verbindung stehende Ende des Zugbolzens (3) mit einer, dem Durchmesser einer um die ursprüngliche Durchtrittsöffnung (12) des Klemmelements (1) vergrößernden Sacklochbohrung entsprechend minimal kleiner gewählten Verdickung (13) versehen ist, sodass das Hineinrutschen des Zugbolzens (3) in das erste Klemmelement (1) verhindert wird.

4. Schnellspannklemme nach einem der vorangegangenen Ansprüche,
**dadurch gekennzeichnet, dass**
das Druckstück (17) aus Polyetheretherketon gefertigt ist.

5. Schnellspannklemme nach einem der vorangegangenen Ansprüche,
**dadurch gekennzeichnet, dass**
der O-Ring (18) aus Silikon gefertigt ist.

6. Schnellspannklemme nach einem der vorangegangenen Ansprüche,
**dadurch gekennzeichnet, dass**
das die Schenkel (4) des ersten Klemmelements (1) eine halbkreisförmige Aussparung (6) begrenzen, welche dem Durchmesser von Rahmenstangen (20) des zu verbindenden Rahmensystems entspricht.

7. Schnellspannklemme nach Anspruch 6,
**dadurch gekennzeichnet, dass**
der Durchmesser der halbkreisförmigen Aussparung etwa 10 % größer als der Durchmesser der Rahmenstange gewählt ist.

8. Schnellspannklemme nach einem der Ansprüche 6 bis 7,
**dadurch gekennzeichnet, dass**
das erste Klemmelement (1) im Bereich der Schenkelbasis (8) eine von der halbkreisförmigen Aussparung (6) weggerichtete schlitzförmige Aussparung (9) aufweist, welche wiederum in einer Vollbohrung (10) endet.

9. Schnellspannklemme nach einem der vorangegangenen Ansprüche,
**dadurch gekennzeichnet, dass**
das Gleitlager (19) aus PEEK besteht.

## Claims

1. A quick clamp for attaching surgical devices like wound hooks or peripheral surgery devices to a frame system comprising two clamping elements (1; 2) provided with a central receiver for a tension bolt (3), wherein the first clamping element (1) is rotatably supported about an axis of the tension bolt (3) and the two clamping elements (1; 2) are mechanically rotatable relative to one another through the tension bolt (3) which is supported by pass-through openings (12) provided in the clamping elements (1; 2) and an eccentric lever is attached through a transversal bolt (16) at an end of the tension bolt (3) that is arranged opposite to the first clamping element (1), wherein the first clamping element (1) to be connected with the frame system includes two arms (4) connected with one another, wherein the second clamping element (2) is configured as a one-component arm (5), whose cross-section includes a quarter-circle recess and an opposite arm is formed by an outer surface section of one of the arms (4) of the first clamping element (1), and a straight bearing (19) is provided on a top side of the first clamping element (1) that is oriented towards the second clamping element (2), wherein the straight bearing is inserted into a recess on the top side, wherein a diameter of the straight bearing (19) essentially corresponds to the cross-section dimensions of the second clamping element (2) and a pressure element (17) is provided between the second clamping element (2) and the eccentric lever (11), wherein the pressure element is pressed into a recess at a top side of the second clamping element (2) and an elastic O-ring (18) is inserted into the recess between the pressure element (17) and the second clamping element (2).

2. The quick clamp according to claim 1, wherein pass-through openings (14) are provided in an arm base (8) of the respective clamping element (1; 2).

3. The quick clamp according to claim 1 or 2, wherein the end of the tension bolt (3), connected with the first clamping element (1), is provided with a thickening (13) that is slightly smaller than a dead hole enlarging the diameter about the original pass-through opening (12) of the clamping element (1) so that the tension bolt (3) is prevented from sliding into the first clamping element (1).

4. The quick clamp according to one of the preceding claims, wherein the pressure element (17) is made from polyether-ketone.

5. The quick clamp according to one of the preceding claims, wherein the O-ring (18) is made from silicon.

6. The quick clamp according to one of the preceding claims, wherein the arms (4) of the first clamping element (1) define a semicircular recess (6) which corresponds to a diameter of frame rods (20) of the frame system to be connected.

7. The quick clamp according to claim 6, wherein the diameter of the semicircular recess is selected approximately % greater than the diameter of the frame rod.

8. The quick clamp according to one of the claims 6 through 7 wherein the first clamping element (1) includes a slot shaped recess (9) in the portion of the arm base, wherein the slot shaped recess is oriented away from the semicircular recess (6) and terminates in a circular borehole (10).

9. The quick clamp according to one of the preceding claims, wherein the straight bearing (19) is made from PEEK.

## Revendications

1. Pince à serrage rapide pour l'application d'auxiliaires chirurgicaux comme des écarteurs pour plaies ou des moyens techniques opératoires périphériques sur un système de cadre, comprenant deux éléments de pinces (1 ; 2) dotés d'un logement central pour un goujon de traction (3), dans laquelle
le premier élément de pince (1) est monté en rotation autour de l'axe du goujon de traction (3), et
les deux éléments de pinces (1 ; 2) sont en interaction en rotation mécanique l'un par rapport à l'autre, au moyen du goujon de traction (3) qui est guidé dans des ouvertures traversantes (12) ménagées dans les éléments de pinces (1 ; 2), et un levier excentrique est monté au moyen d'un goujon transversal (16) à l'extrémité, opposée à l'élément de pince (1), du goujon de traction (3),
dans laquelle le premier élément de pince (1) à relier au système de cadre comporte deux branches (4) reliées l'une à l'autre,
**caractérisée en ce que**
le second élément de pince (2) est réalisé sous la forme d'une branche d'une seule pièce (5), dont la section transversale présente une échancrure en forme de quart de cercle, et la branche antagoniste est formée par un tronçon de surface extérieure de l'une des branches (4) du premier élément de pince (1),
et sur la face supérieure, orientée vers le second élément de pince (2), du premier élément de pince (1), il est prévu un palier coulissant (19) introduit dans un évidement sur la surface supérieure, tel que le diamètre du palier coulissant (19) correspond essentiellement aux dimensions de section transversale du second élément de pince (2), et entre le second élément de pince (2) et le levier excentrique (11) est montée une pièce de pression (17), qui est enfoncée dans un évidement à la surface supérieure du second élément de pince (2), et une bague torique à effet ressort (18) est introduite dans l'évidement entre la pièce de pression (17) et le second élément de pince (2).

2. Pince à serrage rapide selon la revendication 1,
**caractérisée en ce que** les ouvertures traversantes (14) sont ménagées dans la base des branches (8) de l'élément de pince respectif (1 ; 2).

3. Pince à serrage rapide selon la revendication 1 ou 2,
**caractérisée en ce que** l'extrémité du goujon de traction (3) qui est en liaison avec le premier élément de pince (1) est pourvu d'un épaississement (13) choisi de façon minimale plus petit en correspondance du diamètre d'un perçage borgne qui agrandit l'ouverture traversante d'origine (12) de l'élément de pince (1),
de sorte qu'un glissement du goujon de traction (3) entrant dans le premier élément de pinces (1) est empêché.

4. Pince à serrage rapide selon l'une des revendications précédentes,
**caractérisée en ce que** la pièce de pression (17) est fabriquée en polyétheréthercétone.

5. Pince à serrage rapide selon l'une des revendications précédentes,
**caractérisée en ce que** la bague torique (18) est réalisé en silicone.

6. Pince à serrage rapide selon l'une des revendications précédentes,
**caractérisée en ce que** les branches (4) du premier élément de pince (1) délimitent un évidement en forme de demi-cercle (6) qui correspond au diamètre des tiges de cadre (20) du système de cadre à relier.

7. Pince à serrage rapide selon la revendication 6,
**caractérisée en ce que** le diamètre de l'évidement en forme de demi-cercle est choisi environ 10 % plus grand que le diamètre des tiges de cadre.

8. Pince à serrage rapide selon l'une des revendications 6 à 7,
**caractérisée en ce que** le premier élément de pince (1) comporte, dans la zone de la base des branches (8), un évidement (9) en forme de fente orienté en éloignement de l'évidement en forme de demi-cercle (6), l'évidement en forme de fente se terminant à son tour dans un perçage complet (10).

9. Pince à serrage rapide selon l'une des revendications précédentes,
**caractérisée en ce que** le palier coulissant (19) est en polyétheréthercétone (PEEK).
